# EUROPÄISCHE PATENTANMELDUNG

(11) **EP 0 701 814 A2**
(43) Veröffentlichungstag der Anmeldung: **20.03.1996**
(21) Anmeldenummer: 95112426.2
(22) Anmeldetag: 08.08.1995
(51) Int. Cl.: A61K 7/48, A61K 7/42

(54) **Kosmetische oder dermatologische Zubereitungen mit einem Gehalt an hydrophobierten anorganischen Pigmenten zum Erhalt des Urocaninsäurestatus der Haut**

(30) Priorität: 19.08.1994 DE 4429468
(71) Anmelder: Beiersdorf Aktiengesellschaft, D-20245 Hamburg (DE)
(72) Erfinder: Gers-Barlag, Heinrich, Dr., D-25495 Kummerfeld (DE); Schulz, Sabine, D-22529 Hamburg (DE); Uhlmann, Beate, Dr., D-22453 Hamburg (DE); Hintze, Ulrich, Dr., D-22459 Hamburg (DE); Schmucker, Robert, Dr., D-22457 Hamburg (DE)

(57) **Zusammenfassung**

Verwendung eines oder mehrerer hydrophobierter, pharmazeutisch bzw. kosmetisch akzeptabler anorganischer Pigmente in kosmetischen oder dermatologischen Zubereitungen zur Verhinderung
- des durch Einwirkung von Wasser hervorgerufenen Aus- oder Abwaschens der hauteigenen cis- bzw. trans-Urocaninsäure von oder aus der menschlichen Haut oder
- des durch Einwirkung von Wasser hervorgerufenen Aus- oder Abwaschens von künstlich auf die Haut aufgetragener cis- bzw. trans-Urocaninsäure von oder aus der menschlichen Haut.

## Beschreibung

Die vorliegende Erfindung betrifft kosmetische und dermatologische Zubereitungen mit einem Gehalt an hydrophobierten anorganischen Pigmenten. In manchen Ausführungsformen betrifft die vorliegende Erfindung auch kosmetische Reinigungsmittel. In weiteren Ausführungsformen betrifft die vorliegende Erfindung Zubereitungen zum Schutze der Haut gegen UV-Strahlung. Ferner betrifft die Erfindung dermatologische Zubereitungen, die den Immunstatus der menschlichen Haut erhalten und verbessern. Schließlich betrifft die Erfindung Zubereitungen, die den Verlust der Haut von bestimmten hauteigenen Inhaltsstoffen bei der Körperwäsche reduzieren oder verhinden.

Unter Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Zeichen der Hautalterung verzögern.

cis-Urocaninsäure (auch cis-Urocansäure oder cis-4-Imidazolylacrylsäure genannt) ist durch folgende Strukturformel gekennzeichnet: 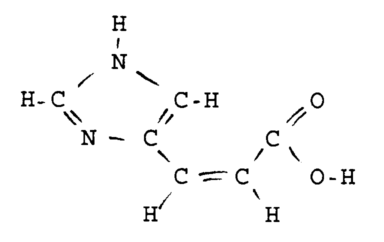
Sie hat die Summenformel C₆H₆N₂O₂ und die Molekularmasse 138,12. cis-Urocaninsäure entsteht beispielsweise durch UV-Bestrahlung des trans-Isomeren, welches in der menschlichen Haut und auch im Schweiß vorkommt.

Die trans-Urocaninsäure ist durch folgende Strukturformel gekennzeichnet: 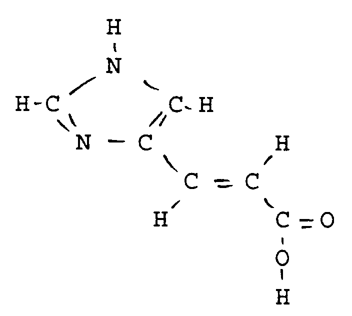
Die trans-Urocaninsäure hat die Summenformel C₆H₆N₂O₂ und die Molekularmasse 138,12 und kommt in der menschlichen Haut und auch im Schweiß vor.

Wenn im Rahmen der hiermit vorgelegten Offenbarung der Begriff "Urocaninsäure" ohne einen Hinweis auf das betreffende Isomere angewandt wird, so werden davon sowohl das cis- als auch das trans-Isomere sowie beliebige Mischungen beider Isomeren erfaßt.

Die deutsche Offenlegungsschrift 41 21 030 zeigt, daß Urocaninsäure antiphlogistisch wirkt, die Folgen allergischer Reaktionen mildert und in hohem Maße allergischen Reaktionen vorbeugt.

Aufgrund antiphlogistischer und antiallergischer Potenz ist die Urocaninsäure wirksam gegen Psoriasis, Neurodermitis und Kontaktdermatitis und Autoimmunkrankheiten, wie z.B. Vitiligo, Pruritus, Alopecia areata, Ichthyose sowie Atopie, bei denen ein ähnlicher Wirkmechanismus vorliegt.

Die herkömmlichen Zubereitungen konnten nicht verhindern, daß die hauteigene Urocaninsäure beim Kontakt mit Wasser und/oder Tensiden oder beim Schwitzen aus- oder abgewaschen wird. Auch herkömmliche Zubereitungen mit einem Gehalt an Urocaninsäure konnten den Urocaninsäurestatus der Haut bestenfalls ein wenig auffrischen, den ursprünglichen Zustand selbst aber nicht mehr erreichen. Dieser war bisher nur nach der individuellen Regenerationszeit des Betroffenen erreichbar.

Zubereitungen zur Pflege der Haut liegen meist in Form von Crèmes, Lotionen, Milchen, Salben, Salbengrundlagen, Ölen, Tinkturen, Stiften, Spray-Formulierungen und dergleichen vor.

Gebräuchliche kosmetische Zubereitungen beispielsweise sind Sonnenschutzmittel. Die Verwendung der trans-Urocaninsäure als Sonnenschutzmittel ist ebenfalls bekannt.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen der Haut.

Als ein Maximum der Erythemwirksamkeit des Sonnenlichtes wird der engere Bereich um 308 nm angesehen.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Wellenlängenbereich zwischen etwa 320 und 400 nm, den sogenannten UVA-Bereich, sind UV-Filtersubstanzen wichtig, da auch solche Strahlen Schäden hervorrufen können. So ist erwiesen, daß UVA-Strahlung zu einer Schädigung der elastischen und kollagenen Fasern des Bindegewebes führt, was die Haut vorzeitig altern läßt und als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Haut- und Zellmetabolismus eingreifen.

Vorwiegend handelt es sich bei solchen photochemischen Reaktionsprodukten um radikalische Verbindungen, z.B. Hydroxyradikale, Hydroperoxyradikale sowie Superoxidionen. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls, kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Obwohl es durchaus vorteilhafte kosmetische bzw. dermatologische Zubereitungen zum Schutze der Haut vor den schädlichen Folgen der Einwirkung von UV-Licht gibt, ist ein oft beobachteter Nachteil, daß die Zubereitungen nicht oder nicht hinreichend wasserfest sind.

Lichtschutzzubereitungen werden besonders häufig an Badestränden bzw. in Freibädern benötigt und angewandt. Wünschenswert ist dann, daß die Lichtschutzformulierung weitgehend wasserfest ist, daß sie also nicht oder nur in geringem Maße von der Haut abgewaschen wird.

Höhere Lichtschutzfaktoren, also etwa solche die oberhalb von LF 15 angesiedelt sind, lassen sich im allgemeinen nur durch hohe Mengen an UV-Filtersubstanzen erreichen. Soll ein Sonnenschutzprodukt auch nach dem Baden noch einen hohen Lichtschutzfaktor aufweisen, muß insbesondere die UV-Filtersubstanz auf der Haut erhalten bleiben.

Es ist an sich schon lästig, wenn nach dem Baden das Sonnenschutzprodukt erneut aufgetragen werden muß. Beim Baden selbst kann die Verwendung einer abwaschbaren Lichtschutzformulierung unter Umständen sogar leichtsinnig und schädlich für die Haut sein, da Wasser das Licht im UVA- und UVB-Bereich schlecht absorbiert, infolgedessen keinen nennenswerten UV-Schutz darstellt, nicht einmal für untergetauchte Hautbereiche.

Für wasserfeste Lichtschutzformulierungen verwendet der Stand der Technik üblicherweise nichtwasserlösliche UV-Filtersubstanzen, wasserabweisende Rohstoffe (z.B. Siliconöle in hohen Konzentrationen) und/oder Filmbildner, insbesondere hochmolekulare Verbindungen (z.B. PVP/Hexadecen-Copolymere). Dabei werden Barrieren zwischen den auf der Haut aufliegenden UV-Filtersubstanzen und dem Wasser aufgebaut.

Nachteilig dabei ist, daß die Diffusion der Filtersubstanzen ins Wasser zwar verzögert, aber nicht vollständig verhindert werden kann. Deshalb können derartige Produkte bei längerem Baden beachtlich an Schutzwirkung verlieren. Aber selbst durch normale Schweißentwicklung bzw. Abwischen dieses Schweißes und der darin gelösten bzw. angelösten Lichtschutzsubstanzen, insbesondere der hauteigenen, aber auch der künstlich aufgetragenen Urocaninsäure, kann erhebliche Verminderung des Lichtschutzes eintreten.

Kann schon durch tensidfreies Wasser der Gehalt der Haut an Urocaninsäure vermindert werden, so ist dies umso ausgeprägter der Fall bei einem zusätzlichen Gehalt an Tensiden, also in Gegenwart von Reinigungsforulierungen.

Bei kosmetischen Reinigungszubereitungen handelt es sich hauptsächlich um oberflächenaktive Substanzen oder Stoffgemische, die dem Verbraucher in verschiedenen Zubereitungen angeboten werden. Zubereitungen solcher Art zeichnen sich im allgemeinen durch einen mehr oder weniger hohen Wassergehalt aus, können aber auch, beispielsweise als Konzentrat, praktisch wasserfrei vorliegen.

Im allgemeinen unterscheiden sich Präparate, welche für das Duschbad vorgesehen sind, nicht oder kaum von Wannenbadzubereitungen, abgesehen davon, daß bei Duschzubereitungen Produkte höherer Viskosität bevorzugt werden, die nicht nach Entnahme aus dem Behälter aus der Hand rinnen. Dies ist bei Wannenbadzubereitungen weniger von praktischer Bedeutung.

Schon bei einem einfachen Wasserbade ohne Zusatz von Tensiden kommt es zunächst zu einer Quellung der Hornschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab- bzw. ausgewaschen. Durch hauteigene tensioaktive Stoffe bewirkt, werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze nach verstärkt werden kann. Insbesondere die hauteigene Urocaninsäure kann aufgrund ihrer hohen Hydrophilie leicht aus der Haut ausgewaschen werden.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden.

Reinigungszubereitungen sind an sich bekannt. Es handelt sich dabei im wesentlichen um oberflächenaktive Substanzen oder Stoffgemische, die dem Verbraucher in verschiedenen Zubereitungen angeboten werden.

Zubereitungen dieser Art sind beispielsweise Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder und dergleichen.

Oberflächenaktive Stoffe - am bekanntesten die Alkalisalze der höheren Fettsäuren, also die klassischen Seifen - sind amphiphile Stoffe, die organische unpolare Substanzen in Wasser emulgieren können.

Diese Stoffe schwemmen nicht nur Schmutz von Haut und Haaren, sie reizen, je nach Wahl des Tensids oder des Tensidgemisches, Haut und Schleimhäute mehr oder minder stark.

Der Stand der Technik kennt allerdings auch wasserarme oder wasserfreie Ölbadzubereitungen verschiedener Art, wobei die Eigenschaften der Fett- oder Ölphase durch Zugabe von oberflächenaktiven Substanzen variiert werden kann. Dabei können je nach Art und Menge der gewählten Bestandteile Zubereitungen formuliert werden, die auf der Badewasseroberfläche entweder spreitende Ölfilme, Öl-in-Wasser-Systeme oder auch Totalsolubilisate ergeben. Schäumende, aber auch wenig schäumende oder nicht schäumende Formulierungen sind möglich.

Im allgemeinen beschränkt sich die Funktionalität derartiger Zubereitungen bei Ölbad- oder Ölcrèmebadzubereitungen auf die Rückfettung oder Überfettung der obersten Hautschichten. Aber auch wirkstoffhaltige Zubereitungen sind bekannt.

Eine Aufgabe der vorliegenden Erfindung war es also, all diesen Übelständen Abhilfe zu schaffen. Aufgabe der Erfindung war es insbesondere, Zubereitungen zur Verfügung zu stellen, welche, gleichgültig, ob ihnen ein zusätzlicher Gehalt an Urocaninsäure beigemischt wird oder nicht, gewährleisten, daß nach Kontakt mit Wasser der Urocaninsäurestatus der Haut möglichst wenig beeinträchtigt, oder, im Falle eines akuten Mangels an Urocaninsäure, ein nahezu physiologischer Urocaninsäurestatus erreicht wird.

Erstaunlicherweise werden diese Aufgaben gelöst durch die
Verwendung eines oder mehrerer hydrophobierter, pharmazeutisch bzw. kosmetisch akzeptabler anorganischer Pigmente in kosmetischen oder dermatologischen Zubereitungen zur Verhinderung
- des durch Einwirkung von Wasser hervorgerufenen Aus- oder Abwaschens der hauteigenen cis- bzw. trans-Urocaninsäure von oder aus der menschlichen Haut oder
- des durch Einwirkung von Wasser hervorgerufenen Aus- oder Abwaschens von künstlich auf die Haut aufgetragener cis- bzw. trans-Urocaninsäure von oder aus der menschlichen Haut.

Erfindungsgemäß ist vorteilhaft, urocaninsäurefreie Zubereitungen zu wählen. Es kann aber gegebenenfalls auch vorteilhaft sein, in erfindungsgemäße Zubereitungen einen Gehalt an cis- und/oder trans-Urocaninsäure einzusetzen.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Zwar beschreiben M.Schmidt und S.G.Steinemann in "XPS studies of amino acids adsorbed on titanium dioxide surfaces", Fresenius' Journal of Analytical Chemistry (1991) 341, S.412 - 415, daß gewisse Aminosäuren leicht von der Oberfläche von Titandioxidpartikeln adsorbiert werden. Ein Hinweis auf die vorliegende Erfindung und ihre vorteilhaften Eigenschaften liefert diese Arbeit jedoch keineswegs. Zudem ist das TiO₂, welches a.a.O. zitiert wird, nicht hydrophob.

Ferner ist überraschend, daß gerade hydrophobierte anorganische Pigmente die erfindungsgemäßen vorteilhaften Eigenschaften aufweisen, da die Urocaninsäure durchaus als hydrophile Substanz zu bezeichnen ist.

Schließlich ist es überraschend, daß es für praktische Zwecke unerheblich ist, ob die erfindungsgemäßen Zubereitungen in bezug auf die Formulierung selbst als "wasserfest" (also beispielsweise unter Verwendung eines ausgeprägten Gehaltes an wasserunlöslichen Filmbildnern) oder als "nicht wasserfest" (also beispielsweise ohne einen solchen Gehalt) zu gelten haben. Der Urocaninsäurestatus der Haut ist erfindungsgemäß ohne Verwendung von filmbildenden Zubereitungen und nach provokativer Wasseranwendung kaum schlechter als bei solcher Verwendung von filmbildenden Zubereitungen.

Erfindungsgemäße kosmetische und dermatologische Zubereitungen enthalten bevorzugt anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂.

Voraussetzung für die Verwendbarkeit anorganischer Pigmente für die erfindungsgemäßen Zwecke ist natürlich die kosmetische bzw. dermatologische Unbedenklichkeit der zugrundeliegenden Substanzen.

Im wesentlichen unerheblich für die vorliegende Erfindung ist dabei, in welchen Modifikationen solche Metalloxide vorliegen. TiO₂ beispielsweise kommt in der Natur in drei Hauptmodifikationen (Rutil, Anatas und Brookit) vor, welche grundsätzlich alle gleichermaßen geeignet sind. Ähnliches gilt für die Modifikationen der Eisenoxide usw.

Vorteilhaft ist, den Partikeldurchmesser der verwendeten Pigmente kleiner als 100 nm zu wählen.

Erfindungsgemäß liegen die anorganischen Pigmente in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

n TiO₂ + m (RO)₃Si-R' -> n TiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen T 805 (DEGUSSA) oder M 262 (KEMIRA) oder M 160 (KEMIRA) oder MT 100 T (TAYCA) erhältlich.

Vorteilhafte SiO₂-Pigmente können aus der Reihe der unter den Handelsbezeichnungen AEROSIL (DEGUSSA) vertriebenen hydrophoben Pigmente, beispielsweise AEROSIL R 812 oder AEROSIL R 972, gewählt werden.

Die Herstellung erfindungsgemäßer Zubereitungen geschieht nach den üblichen, dem Fachmanne geläufigen Regeln. Vorteilhaft liegen die erfindungsgemäßen Zubereitungen als Emulsionen, bevorzugt O/W-Emulsionen, vor. Es ist aber auch möglich und erfindungsgemäß gegebenenfalls vorteilhaft, andere Formulierungsarten zu wählen, beispielsweise Hydrodispersionen, Gele, Öle, Multiple Emulsionen, beispielsweise in Form von W/O/W- oder O/W/O-Emulsionen, wasserfreie Salben bzw. Salbengrundlagen usw. Ferner stellen Waschzubereitungen wie beispielsweise Shampoos, insbesondere aber Ölbäder, Ölcrèmebäder und Duschöle äußerst vorteilhafte Verkörperungen der vorliegenden Erfindung dar.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. In einer multiplen Emulsion (zweiten Grades) hingegen sind in solchen Tröpfchen feiner disperse Tröpfchen der ersten Phase emulgiert. Auch in diesen Tröpfchen wiederum können noch feiner disperse Tröpfchen vorliegen (multiple Emulsion dritten Grades) und so fort.

So wie man also bei den einfachen Emulsionen von W/O- oder O/W-Emulsionen spricht (Wasser-in-Oel oder Oel-in-Wasser), gibt es bei multiplen Emulsionen W/O/W-, O/W/O-, O/W/O/W-, W/O/W/O-Emulsionen und so fort.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Es ist möglich und vorteilhaft, das oder die hydrophoben anorganischen Pigmente und gewünschtenfalls die Urocaninsäure zu jedem beliebigen Zeitpunkte der Emulsionsherstellung dem Emulsionsgemisch zuzugeben. Dabei können Pigment oder Pigmente gewünschtenfalls die Urocaninsäure sowohl getrennt als auch bereits miteinander vereinigt dem Emulsionsgemisch zugegeben werden.

Besonders vorteilhafte Zubereitungen werden ferner erhalten, wenn die erfindungsgemäßen Wirkstoffe mit Antioxidantien kombiniert werden. Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden.

Besonders vorteilhaft werden die Antioxidantien gewählt aus der Gruppe bestehend aus

Aminosäuren (z.B. Glycin, Histidin, Tyrosin, Tryptophan) und deren Derivate, Imidazole und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Furalglucitol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist gewünschtenfalls vorteilhaft, den erfindungsgemäßen Zubereitungen Urocaninsäure einzuverleiben, dann vorzugsweise in Konzentrationen von 0,00001 mg/ml - 60 mg/ml, bezogen auf das Gesamtvolumen der Zubereitungen. Bevorzugt sind Zubereitungen mit Konzentrationen von 0,01 mg/ml - 2,0 mg/ml, insbesondere von 0,05 mg/ml - 1,0 mg/ml, jeweils bezogen auf das Gesamtvolumen der Zubereitungen.

Vorteilhaft liegen die erfindungsgemäßen urocaninsäurehaltigen Formulierungen als Emulsionen, bevorzugt O/W-Emulsionen, vor. Es ist aber auch möglich und erfindungsgemäß gegebenenfalls vorteilhaft, andere Formulierungsarten zu wählen, beispielsweise Hydrodispersionen, Gele, Öle, multiple Emulsionen, beispielsweise in Form von W/O/W- oder O/W/O-Emulsionen, wasserfreie Salben bzw. Salbengrundlagen usw. Ferner stellen Waschzubereitungen wie beispielsweise Shampoos, Ölbäder, Ölcrèmebäder und Duschöle äußerst vorteilhafte Verkörperungen der vorliegenden Erfindung dar. Es hat sich ferner als günstig erwiesen, wäßrige oder alkoholisch/wäßrige oder alkoholische oder acetonisch/wäßrige oder acetonische oder acetonisch/alkoholische Lösungen der Urocaninsäure in die Formulierungen einzuarbeiten.

Es ist ferner von Vorteil, den Zusammensetzungen Hilfs- und/oder Zusatzstoffe einzuverleiben, die die Stabilität der Urocaninsäure bzw. deren Derivate erhöhen oder die aus galenischer Sicht die Qualität der Zusammensetzungen verbessern oder verändern.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhaft können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung.

Der pH-Wert der Erfindungsgemäßen Zubereitungen kann vorteilhaft im sauren Bereich eingestellt werden, wobei der pH-Bereich von 3,5 - 7 bevorzugt wird, besonders bevorzugt von 4 - 5.

Erfindungsgemäße Zubereitungen können auch vorteilhaft als Sonnenschutzmittel dienen. Ferner ist es von hohem Vorteil, erfindungsgemäße Zubereitungen bei allen sportlichen Aktivitäten, namentlich schweißtreibenden, zu verwenden.

Emulsionen gemäß der Erfindung z.B. in Form einer Sonnenschutzcreme oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die UVB-Filter können öllöslich oder wasserlöslich sein. Vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Vorteilhaft können die erfindungsgemäßen Zubereitungen außerdem Substanzen enthalten, die UV-Strahlung im UVA-Bereich absorbieren. UVA-Filter, die erfindungsgemäß vorteilhaft verwendet werden können, sind beispielsweise Derivate des Dibenzoylmethans, insbesondere 1-(4'-tert.Butylphenyl)-3-(4'-methoxyphenyl)propanl-1,3-dion und um 1-Phenyl-3-(4'-isopropylphenyl)propan-1,3-dion.

Die Gesamtmenge der UVA-Filtersubstanzen kann vorteilhaft 0,1 Gew.-% bis 30 Gew.-%, vorzugsweise 0,5 bis 10 Gew.-%, insbesondere 1 bis 6 Gew.-% betragen, bezogen auf das Gesamtgewicht der Zubereitung.

Vorteilhaft sind auch solche kosmetische und dermatologische Zubereitungen, die in der Form eines Sonnenschutzmittels, eines Pré-Soleil- oder Après-Soleil-Produktes vorliegen. Vorteilhaft enthalten diese zusätzlich mindestens einen UVA-Filter und/oder mindestens einen UVB-Filter.

Ferner sind auch solche kosmetische und dermatologische Zubereitungen besonders vorteilhaft, die in der Form eines Sonnenschutzmittels, eines Pré-Soleil- oder Après-Soleil-Produktes vorliegen und zusätzlich zu dem oder den UVA-Filtern und/oder dem oder den UVB-Filtern ein oder mehrere Antioxidantien enthalten.

Die Gesamtmenge der Antioxidantien kann vorteilhaft 0,1 Gew.-% bis 30 Gew.-%, vorzugsweise 0,5 bis 10 Gew.-%, insbesondere 1 bis 6 Gew.-% betragen, bezogen auf das Gesamtgewicht der Zubereitung.

Erfindungsgemäße kosmetische Reinigungsmittel können anionische, nichtionische und/oder amphotere Tenside enthalten, beispielsweise herkömmliche Seifen wie Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate.

Erfindungsgemäß vorteilhaft sind Reinigungszubereitungen in Form eines Ölbades, eines Ölcrèmebades oder, besonders vorteilhaft, eines Duschöls. Solche Zubereitungen zeichnen sich dadurch aus, daß sie im wesentlichen aus Ölkomponenten und Tensiden bestehen und im wesentlichen wasserfrei sind oder nur geringe Wasseranteile besitzen.

Bevorzugte Duschöle zeichnen sich aus durch einen Gehalt von höchstens 55 Gew.-%, bezogen auf das Gesamtgewicht der Zubereitungen, eines oder mehrerer Tenside, gewählt aus der Gruppe Fettalkoholethoxylate, Fettalkoholsulfate, Amide der Fettalkoholsulfate, Fettalkoholethersulfate, Amide der Fettalkoholethersulfate, Fettsäuremonoethanolamide, Fettsäurediethanolamide, sowie einen Gehalt von mindestens 45 Gew.-%, bezogen auf das Gesamtgewicht der Zubereitungen, eines oder mehrerer Ölkomponenten, gewählt aus der Gruppe der Öle mit einem hohen Gehalt an Triglyceriden gesättigter und/oder ungesättigter, verzweigter und/oder unverzweigter Fettsäuren, oder ausschließlich solche Triglyceride enthaltend, enthaltend ferner hydrophobierte anorganische Pigmente und gegebenenfalls weitere kosmetische oder pharmazeutische Hilfs-, Zusatz- und/oder Wirkstoffe, wobei die Zubereitungen dann vorzugsweise im wesentlichen wasserfrei sind,

Vorteilhaft weisen die erfindungsgemäß günstig zu verwendenden Fettalkoholsulfate bzw. Fettalkoholethersulfate folgende Struktur auf: 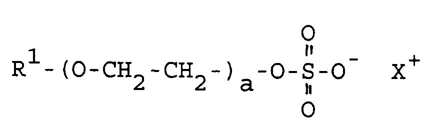
Dabei kann a Werte von 0 bis 10, vorteilhaft 1 bis 5 annehmen. R¹ wird gewählt aus der Gruppe der verzweigten und unverzweigten Alkylgruppen mit 6 bis 24 Kohlenstoffatomen.

X⁺ wird gewählt aus der Gruppe der Alkaliionen sowie der Gruppe der mit einer oder mehreren Alkyl- und/oder mit einer oder mehreren Hydroxyalkylresten substituierten Ammoniumionen.

Vorteilhaft weisen die erfindungsgemäß günstig zu verwendenden Amide der Fettalkoholsulfate bzw. der Fettalkoholethersulfate folgende Struktur auf: 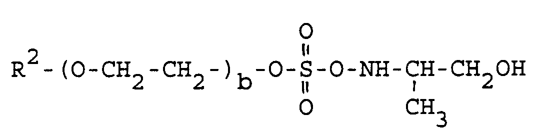
Dabei kann b Werte von 0 bis 10, vorteilhaft 1 bis 5 annehmen. R² wird gewählt aus der Gruppe der verzweigten und unverzweigten Alkylgruppen mit 6 bis 24 Kohlenstoffatomen.

Bevorzugtes Fettalkoholethersulfat ist MIPA-Laurethsulfat.

Vorteilhaft weisen die erfindungsgemäß günstig zu verwendenden Fettalkoholethoxylate folgende Struktur auf:

R³-(O-CH₂-CH₂-)_{c}-OH

Dabei kann c Werte von 1 bis 45 annehmen, bevorzugt von 1 bis 10. R³ wird gewählt aus der Gruppe der verzweigten und unverzweigten Alkylgruppen mit 6 bis 24 Kohlenstoffatomen.

Bevorzugtes Fettalkoholethoxylat ist Laureth-4.

Vorteilhaft weisen die erfindungsgemäß günstig zu verwendenden Fettsäuremono- bzw. -diethanolamide folgende Strukturen auf: 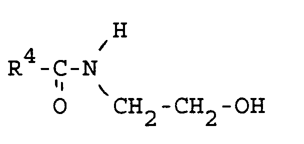
bzw. 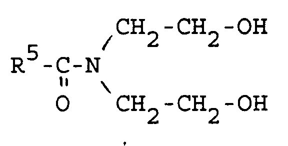
R⁴ bzw. R⁵ werden dabei gewählt aus der Gruppe der verzweigten und unverzweigten Alkylgruppen und/oder Alkenylgruppen mit 6 bis 24 Kohlenstoffatomen.

Bevorzugtes Fettsäurediethanolamid ist Kokosfettsäurediethanolamid (Cocamide DEA). Natürliche Kokosfettsäure enthält als wesentliche Bestandteile Laurinsäure zu 44 - 51 Gew.-%, Myristinsäure zu 13 - 18 Gew.-%, Palmitinsäure zu 8 - 10 Gew.-%, Caprylsäure zu 6 - 9 Gew.-%, Caprinsäure zu 6 - 10 Gew.-%, Ölsäure zu 5 - 8 Gew.-%, Stearinsäure zu 1 - 3 Gew.-%, Linolsäure zu 0 - 2 Gew.-% und Capronsäure zu 0 - 1 Gew.-%.

Ganz besonders bevorzugt ist, Gemische aus MIPA-Laurethsulfat, Laureth-4 und Kokosfettsäurediethanolamid einzusetzen. Solche Gemische sind beispielsweise unter der Bezeichnung ZETESOL^{(R)} 100 von der Firma Zschimmer & Schwarz Chemische Fabriken, Lahnstein/Rhein, oder TEXAPON^{(R)} WW 99 von der Henkel KGaA, Düsseldorf, erhältlich.

Die erfindungsgemäßen Öle werden vorzugsweise gewählt aus der Gruppe der Triglyceride folgender Struktur: 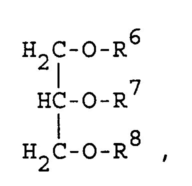
wobei R⁶, R⁷ und R⁸ unabhängig voneinander gewählt werden aus der Gruppe der verzweigten und unverzweigten, Alkylcarboxyl- bzw. Alkenylcarboxylgruppen mit 12 bis 24 Kohlenstoffatomen. Es ist gegebenenfalls vorteilhaft, wenn eine oder mehrere aliphatische Wasserstoffatome der Alkylcarboxyl- bzw. Alkenylcarboxylgruppen durch Hydroxylgruppen substituiert sind.

Insbesondere ist vorteilhaft, wenn R⁶, R⁷ und/oder R⁸ 16 bis 20 Kohlenstoffatome aufweisen und aus der Gruppe der einfach bis dreifach ungesättigten Carbonsäurereste gewählt werden.

Wenn R⁶, R⁷ und/oder R⁸ Hydroxylgruppen tragen, ist der bevorzugte Alkenylcarboxylrest der Ricinolsäurerest.

Besonders vorteilhaft ist, die erfindungsgemäßen Öle aus der Gruppe Sojaöl, Sonnenblumenöl, Weizenkeimöl und Ricinusöl zu wählen.

Bevorzugte Zubereitungen enthalten 0 bis 60 Gew.-% Sojaöl und 0 bis 60 Gew.-% Weizenkeimöl und 0 bis 60 Gew.-% Sonnenblumenöl, mit der Maßgabe, daß die Summe der Einzelkonzentrationen dieser Öle 30 - 60 Gew.-% ausmacht, ferner Rizinusöl in einer Konzentration von 5 - 25 Gew.-%, wobei diese Konzentrationen jeweils bezogen sind auf das Gesamtgewicht der Zubereitungen.

Die Duschöle enthalten außer den vorgenannten Tensiden gegebenenfalls die in der Kosmetik bzw. der pharmazeutischen Galenik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Vorteilhaft ist insbesondere, Verdicker aus der Gruppe der Polyoxyethylen-Polyoxypropylen-Blockcopolymere zu wählen. Solche Blockcoploymere sind unter der Bezeichnung "Poloxamere" bekannt und zeichnen sich durch folgende Struktur aus: 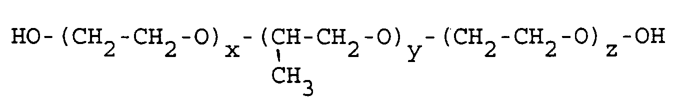
Dabei nimmt x vorteilhaft Werte zwischen 2 und 20 an. y nimmt vorteilhaft Werte zwischen 10 und 50 an. z nimmt vorteilhaft Werte zwischen 2 und 20 an.

Wenn Zubereitungen gemäß der vorliegenden Erfindung außer den erfindungsgemäßen Tensiden weitere Tenside enthalten sollen, so wird bevorzugt, deren Konzentration in bezug auf das Gewicht der Gesamtzusammensetzung nicht größer als 5 Gew.-% zu wählen.

Erfindungsgemäß können als günstige Antioxidantien alle für kosmetische und/oder dermatologische Anwendungen geeigneten oder gebräuchlichen Antioxidantien verwendet werden.

Die Beispiele stellen vorteilhafte Verkörperungen der vorliegenden Erfindung dar:

### Beispiel 1

| Sonnencrème, O/W, Lichtschutzfaktor 20 | |
|---|---|
| | Gew.-% |
| Cyclomethicon | 3,00 |
| Glycerylstearat + PEG-30 Stearat | 2,00 |
| Wollwachsalkohol | 0,10 |
| Glycerylstearat | 3,00 |
| Isopropylpalmitat | 2,00 |
| Octyldodecanol | 1,00 |
| C₁₂₋₁₅Alkylbenzoat | 2,00 |
| Glycerin | 3,00 |
| Cetylalkohol | 3,00 |
| Myristylmyristat | 2,00 |
| Octylmethoxycinnamat | 4,50 |
| Butylmethoxydibenzoylmethan | 2,00 |
| Phenylbenzimidazolsulfonsäure | 3,00 |
| Tocopherylacetat | 0,50 |
| EDTA-Lösung (20 %-ig) | 0,50 |
| NaOH (45 %-ig) | q.s. |
| Ethylalkohol | 4,00 |
| Konservierungsmittel | q.s. |
| Parfüm | q.s. |
| Hydrophobes TiO₂ | 2,00 |
| Wasser, VES | ad 100,00 |

### Beispiel 2

| Sonnencrème, O/W, Lichtschutzfaktor 8 | |
|---|---|
| | Gew.-% |
| Cyclomethicon | 3,00 |
| Glycerylstearat + PEG-30 Stearat | 2,00 |
| Glycerylstearat | 3,00 |
| Isopropylpalmitat | 2,00 |
| Octyldodecanol | 3,00 |
| Glycerin | 3,00 |
| Cetylalkohol | 3,00 |
| Phenylbenzimidazolsulfonsäure | 1,00 |
| Octylmethoxycinnamat | 5,00 |
| Butylmethoxydibenzoylmethan | 0,50 |
| Tocopherylacetat | 0,50 |
| EDTA-Lösung (20 %-ig) | 0,50 |
| NaOH | q.s. |
| Ethylalkohol | 1,50 |
| Konservierungsmittel | q.s. |
| Parfüm | q.s. |
| Hydrophobes TiO₂ | 1,00 |
| Wasser, VES | ad 100,00 |

### Beispiel 3

| Sonnenmilch, O/W | |
|---|---|
| | Gew.-% |
| Trilaureth-4 Phosphat | 0,75 |
| Triceteareth-4 Phosphat | 1,00 |
| Glycerylstearat + PEG-100 Stearat | 1,00 |
| Glycerylstearat + Ceteareth-20 | 0,80 |
| Glyceryllanolat | 0,50 |
| Isopropylpalmitat | 3,00 |
| Glycerin-Caprylat/Caproat ("Caprylic/Capric Triglyceride") | 5,00 |
| Cetylalkohol | 1,00 |
| Xanthangummi | 0,30 |
| Octylmethoxycinnamat | 6,00 |
| Butylmethoxydibenzoylmethan | 0,50 |
| Methylbenzylidencampher | 3,00 |
| Phenylbenzimidazolsulfonsäure | 4,00 |
| Butylhydroxytoluol | 0,06 |
| EDTA-Lösung (20 %-ig) | 0,50 |
| NaOH | q.s. |
| Konservierungsmittel | q.s. |
| Hydrophobes TiO₂ | 3,00 |
| Wasser, VES | ad 100,00 |

### Beispiel 4

| Sonnenmilch, O/W | |
|---|---|
| | Gew.-% |
| Cyclomethicon | 2,00 |
| Cetearylalkohol + PEG-40-hydriertes Rizinusöl + Natriumcetearylsulfat | 2,50 |
| Glyceryllanolat | 1,00 |
| Glycerin-Caprylat/Caproat ("Caprylic/Capric Triglyceride") | 2,00 |
| Octylstearat | 3,00 |
| Rizinusöl | 4,00 |
| Glycerin | 3,00 |
| Carbomer | 0,23 |
| Hydroxypropylmethylcellulose | 0,30 |
| PVP/Hexadecen Copolymer | 1,50 |
| Octylmethoxycinnamat | 4,00 |
| Butylmethoxydibenzoylmethan | 2,00 |
| Methylbenzylidencampher | 3,50 |
| Butylhydroxytoluol | 0,03 |
| EDTA-Lösung (20 %-ig) | 0,50 |
| NaOH | q.s. |
| Konservierungsmittel | q.s. |
| Parfüm | q.s. |
| Hydrophobes TiO₂ | 1,50 |
| Wasser, VES | ad 100,00 |

### Beispiel 5

| Sonnenmilch, O/W | |
|---|---|
| | Gew.-% |
| Stearinsäure | 2,00 |
| Cetylpalmitat | 1,00 |
| Glycerin-Caprylat/Caproat ("Caprylic/Capric Triglyceride") | 2,00 |
| Sorbitanmonooleat | 1,00 |
| Sorbitanmonostearat | 1,50 |
| Paraffinöl, DAB 9 | 1,29 |
| Cetearylalkohol | 0,80 |
| Glycerin | 3,00 |
| 2-Hydroxy-4-methoxybenzophenon | 1,00 |
| Octylmethoxycinnamat | 5,00 |
| Methylbenzylidencampher | 1,00 |
| Butylhydroxytoluol | 0,06 |
| Simethicon | 0,20 |
| Polyethylenglycol-150 | 3,00 |
| Triethanolamin | 0,85 |
| Carbomer | 0,10 |
| Ethylalkohol | 3,00 |
| Konservierungsmittel | q.s. |
| Parfüm | q.s. |
| Hydrophobes TiO₂ | 2,00 |
| Wasser, VES | ad 100,00 |

### Beispiel 6

| Sonnencrème, O/W, Lichtschutzfaktor 20 | |
|---|---|
| | Gew.-% |
| Cyclomethicon | 3,00 |
| Glycerylstearat + PEG-30 Stearat | 2,00 |
| Wollwachsalkohol | 0,10 |
| Glycerylstearat | 3,00 |
| Isopropylpalmitat | 2,00 |
| Octyldodecanol | 1,00 |
| C₁₂₋₁₅Alkylbenzoat | 2,00 |
| Glycerin | 3,00 |
| Cetylalkohol | 3,00 |
| Myristylmyristat | 2,00 |
| Octylmethoxycinnamat | 4,50 |
| Butylmethoxydibenzoylmethan | 2,00 |
| Phenylbenzimidazolsulfonsäure | 3,00 |
| Tocopherylacetat | 0,50 |
| EDTA-Lösung (20 %-ig) | 0,50 |
| NaOH (45 %-ig) | q.s. |
| Ethylalkohol | 4,00 |
| Konservierungsmittel | q.s. |
| Parfüm | q.s. |
| Hydrophobes TiO₂ | 2,00 |
| cis-Urocaninsäure | 0,25 |
| Wasser, VES | ad 100,00 |

### Beispiel 7

| Sonnencrème, O/W, Lichtschutzfaktor 8 | |
|---|---|
| | Gew.-% |
| Cyclomethicon | 3,00 |
| Glycerylstearat + PEG-30 Stearat | 2,00 |
| Glycerylstearat | 3,00 |
| Isopropylpalmitat | 2,00 |
| Octyldodecanol | 3,00 |
| Glycerin | 3,00 |
| Cetylalkohol | 3,00 |
| Phenylbenzimidazolsulfonsäure | 1,00 |
| Octylmethoxycinnamat | 5,00 |
| Butylmethoxydibenzoylmethan | 0,50 |
| Tocopherylacetat | 0,50 |
| EDTA-Lösung (20 %-ig) | 0,50 |
| NaOH | q.s. |
| Ethylalkohol | 1,50 |
| Konservierungsmittel | q.s. |
| Parfüm | q.s. |
| Hydrophobes TiO₂ | 1,00 |
| Urocaninsäure | 0,50 |
| Wasser, VES | ad 100,00 |

### Beispiel 8

| Sonnenmilch, O/W | |
|---|---|
| | Gew.-% |
| Trilaureth-4 Phosphat | 0,75 |
| Triceteareth-4 Phosphat | 1,00 |
| Glycerylstearat + PEG-100 Stearat | 1,00 |
| Glycerylstearat + Ceteareth-20 | 0,80 |
| Glyceryllanolat | 0,50 |
| Isopropylpalmitat | 3,00 |
| Glycerin-Caprylat/Caproat ("Caprylic/Capric Triglyceride") | 5,00 |
| Cetylalkohol | 1,00 |
| Xanthangummi | 0,30 |
| Octylmethoxycinnamat | 6,00 |
| Butylmethoxydibenzoylmethan | 0,50 |
| Methylbenzylidencampher | 3,00 |
| Phenylbenzimidazolsulfonsäure | 4,00 |
| Butylhydroxytoluol | 0,06 |
| EDTA-Lösung (20 %-ig) | 0,50 |
| NaOH | q.s. |
| Konservierungsmittel | q.s. |
| Urocaninsäure | 1,00 |
| Hydrophobes TiO₂ | 3,00 |
| Wasser, VES | ad 100,00 |

### Beispiel 9

| Sonnenmilch, O/W | |
|---|---|
| | Gew.-% |
| Cyclomethicon | 2,00 |
| Cetearylalkohol + PEG-40-hydriertes Rizinusöl + Natriumcetearylsulfat | 2,50 |
| Glyceryllanolat | 1,00 |
| Glycerin-Caprylat/Caproat ("Caprylic/Capric Triglyceride") | 2,00 |
| Octylstearat | 3,00 |
| Rizinusöl | 4,00 |
| Glycerin | 3,00 |
| Carbomer | 0,23 |
| Hydroxypropylmethylcellulose | 0,30 |
| PVP/Hexadecen Copolymer | 1,50 |
| Octylmethoxycinnamat | 4,00 |
| Butylmethoxydibenzoylmethan | 2,00 |
| Methylbenzylidencampher | 3,50 |
| Butylhydroxytoluol | 0,03 |
| EDTA-Lösung (20 %-ig) | 0,50 |
| NaOH | q.s. |
| Konservierungsmittel | q.s. |
| Parfüm | q.s. |
| Urocaninsäure | 0,25 |
| Hydrophobes TiO₂ | 1,50 |
| Wasser, VES | ad 100,00 |

### Beispiel 10

| Sonnenmilch, O/W | |
|---|---|
| | Gew.-% |
| Stearinsäure | 2,00 |
| Cetylpalmitat | 1,00 |
| Glycerin-Caprylat/Caproat ("Caprylic/Capric Triglyceride") | 2,00 |
| Sorbitanmonooleat | 1,00 |
| Sorbitanmonostearat | 1,50 |
| Paraffinöl, DAB 9 | 1,29 |
| Cetearylalkohol | 0,80 |
| Glycerin | 3,00 |
| 2-Hydroxy-4-methoxybenzophenon | 1,00 |
| Octylmethoxycinnamat | 5,00 |
| Methylbenzylidencampher | 1,00 |
| Butylhydroxytoluol | 0,06 |
| Simethicon | 0,20 |
| Polyethylenglycol-150 | 3,00 |
| Triethanolamin | 0,85 |
| Carbomer | 0,10 |
| Ethylalkohol | 3,00 |
| Konservierungsmittel | q.s. |
| Parfüm | q.s. |
| Urocaninsäure | 1,50 |
| Hydrophobes TiO₂ | 2,00 |
| Wasser, VES | ad 100,00 |

| Beispiel | 11 | 12 | 13 | 14 |
|---|---|---|---|---|
| Sojaöl | 54,0 | 40,0 | 37,0 | 42,0 |
| Rizinusöl | 14,0 | 14,0 | 9,0 | 14,0 |
| ZETESOL 100 | 30,0 | 51,0 | 51,0 | 41,0 |
| Poloxamer 101 | 2,0 | 2,0 | 2,0 | 2,0 |
| hydrophobes TiO₂ | 2,5 | 2,5 | 2,5 | 2,5 |
| Parfum, Antioxidantien, Konservierungsstoffe | q.s. | q.s. | q.s. | q.s. |

| Beispiel | 15 | 16 | 17 | 18 |
|---|---|---|---|---|
| Weizenkeimöl | 54,0 | 40,0 | 37,0 | 42,0 |
| Rizinusöl | 14,0 | 14,0 | 9,0 | 14,0 |
| ZETESOL 100 | 30,0 | 51,0 | 51,0 | 41,0 |
| Poloxamer 101 | 2,0 | 2,0 | 2,0 | 2,0 |
| hydrophobes TiO₂ | 2,5 | 2,5 | 2,5 | 2,5 |
| Parfum, Antioxidantien, Konservierungsstoffe | q.s. | q.s. | q.s. | q.s. |

| Beispiel | 19 | 20 | 21 | 22 |
|---|---|---|---|---|
| Sonnenblumenöl | 54,0 | 40,0 | 37,0 | 42,0 |
| Rizinusöl | 14,0 | 14,0 | 9,0 | 14,0 |
| ZETESOL 100 | 30,0 | 51,0 | 51,0 | 41,0 |
| Poloxamer 101 | 2,0 | 2,0 | 2,0 | 2,0 |
| hydrophobes TiO₂ | 2,5 | 2,5 | 2,5 | 2,5 |
| Parfum, Antioxidantien, Konservierungsstoffe | q.s. | q.s. | q.s. | q.s. |

### Vergleichsbeispiel 1

| Sonnenmilch, O/W | |
|---|---|
| | Gew.-% |
| Stearinsäure | 2,00 |
| Cetylpalmitat | 1,00 |
| Glycerin-Caprylat/Caproat ("Caprylic/Capric Triglyceride") | 2,00 |
| Sorbitanmonooleat | 1,00 |
| Sorbitanmonostearat | 1,50 |
| Paraffinöl, DAB 9 | 1,29 |
| Cetearylalkohol | 0,80 |
| Glycerin | 3,00 |
| Butylhydroxytoluol | 0,06 |
| Simethicon | 0,20 |
| Polyethylenglycol-150 | 3,00 |
| Triethanolamin | 0,85 |
| Carbomer | 0,10 |
| Ethylalkohol | 3,00 |
| Konservierungsmittel | q.s. |
| Parfüm | q.s. |
| Wasser, VES | ad 100,00 |

### Wirkungsnachweis:

Die Eigenschaften der Sonnenmilch gemäß Beispiel 5 (SM 1) wurden mit denen der TiO₂-freien, ansonsten identischen Zubereitung gemäß Vergleichsbeispiel 1 (SM 2) getestet.

Es wurden Reflexionsspektren vor und nach Behandlung der volaren Unterarme von acht Testpersonen mit den Testpräparaten sowie nach 20-minütigem Baden der Unterarme mittels eines Fluoreszenzspektrometers LS 50 (Perkin Elmer) gemessen. Urocaninsäure zeigt Absorption bei 308 nm; ein niedriger Reflexionspeak bei dieser Wellenlänge deutet demzufolge auf relativ hohe Urocaninsäurekonzentration hin.

Die Zunahme der Höhe des Peaks bei 308 nm nach dem Waschen betrug gegenüber dem "Normalzustande", also gegenüber vor dem Waschen, bei SM 2: 46,3 %, bei SM 1: 29,7 %.

Dies zeigt, daß die auf oder in der Haut verbliebene Urocaninsäurekonzentration bei Verwendung von SM 1 erheblich höher sein muß als bei Verwendung von SM 2.

## Patentansprüche

1. Verwendung eines oder mehrerer hydrophobierter, pharmazeutisch bzw. kosmetisch akzeptabler anorganischer Pigmente in kosmetischen oder dermatologischen Zubereitungen zur Verhinderung
- des durch Einwirkung von Wasser hervorgerufenen Aus- oder Abwaschens der hauteigenen cis- bzw. trans-Urocaninsäure von oder aus der menschlichen Haut oder
- des durch Einwirkung von Wasser hervorgerufenen Aus- oder Abwaschens von künstlich auf die Haut aufgetragener cis- bzw. trans-Urocaninsäure von oder aus der menschlichen Haut.

2. Verwendung nach Anspruch 1, dadurch gekennzeichnet, daß die anorganischen Pigmente auf den Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle bzw. Abmischungen aus solchen Oxiden basieren.

3. Verwendung nach Anspruch 1, dadurch gekennzeichnet, daß das oder die hydrophobierten anorganischen Pigmente erhältlich sind durch Hydrophobierungsverfahren anorganischer Pigmente gemäß DE-OS 33 14 742.

4. Verwendung nach Anspruch 1, dadurch gekennzeichnet, daß die kosmetischen oder dermatologischen Zubereitungen 0,01 Gew.-% bis 30 Gew.-%, insbesondere 0,1 Gew.-% bis 6 Gew.-%, bezogen auf das Gesamtgewicht der Formulierungen, an anorganischen Pigmenten enthalten.

5. Verwendung nach Anspruch 1, dadurch gekennzeichnet, daß die kosmetischen oder dermatologischen Zubereitungen Urocaninsäure enthalten, vorzugsweise in Konzentrationen von 0,00001 mg/ml - 60 mg/ml, bezogen auf das Gesamtvolumen der Zubereitungen, besonders bevorzugt von 0,05 mg/ml - 1,0 mg/ml, jeweils bezogen auf das Gesamtvolumen der Zubereitungen.

6. Verwendung nach Anspruch 1, dadurch gekennzeichnet, daß die kosmetischen Zubereitungen gewählt werden aus der Gruppe der Lichtschutzzubereitungen.

7. Verwendung nach Anspruch 1, dadurch gekennzeichnet, daß die kosmetischen Zubereitungen gewählt werden aus der Gruppe der Ölbäder.
